(19) Europäisches Patentamt
European Patent Office
Office européen des brevets

(11) Veröffentlichungsnummer: **0 052 750**
**B1**

(12) **EUROPÄISCHE PATENTSCHRIFT**

(45) Veröffentlichungstag der Patentschrift: **28.03.84**

(51) Int. Cl.³: **C 07 D 275/06**

(21) Anmeldenummer: **81108480.5**

(22) Anmeldetag: **19.10.81**

# (54) Verfahren zur Herstellung von Saccharin.

(30) Priorität: **24.11.80 DE 3044112**

(43) Veröffentlichungstag der Anmeldung: **02.06.82 Patentblatt 82/22**

(45) Bekanntmachung des Hinweises auf die Patenterteilung: **28.03.84 Patentblatt 84/13**

(84) Benannte Vertragsstaaten: **AT BE CH DE FR GB IT LI NL SE**

(56) Entgegenhaltungen:
**DD - A - 4 564**
**US - A - 4 042 600**

**Ullmanns Enzyklopädie der technischen Chemie 4. Aufl., Bd. 22, S. 356-357**

(73) Patentinhaber: **BASF Aktiengesellschaft, Carl-Bosch-Strasse 38, D-6700 Ludwigshafen (DE)**

(72) Erfinder: **Tonne, Peter, Dr., Triftbrunnenweg 63, D-6730 Neustadt 1 (DE)**
Erfinder: **Jaedicke, Hagen, Dr., Budapester Strasse 49, D-6700 Ludwigshafen (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Saccharin

Die vorliegende Erfindung betrifft ein neues Verfahren zur Herstellung von Saccharin (I)

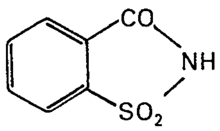

I

Die Herstellung dieser wichtigen, hauptsächlich als Süßstoff verwendeten Verbindung, ist ein vielbearbeitetes Problem, und dementsprechend sind zahlreiche Verfahren und Verfahrensverbesserungen bekannt geworden (vgl. z.B. Ullmanns Enzyklopädie der Technischen Chemie, 3. Auflage, Band 16, 1965, S. 478ff). Trotzdem konnte die klassische Synthese über die Sulfochlorierung von Toluol (Ullmann, cit., S. 479) bisher jedoch praktisch nicht verdrängt werden, obwohl sie verfahrenstechnisch umständlich ist, teure Reagenzien erfordert und überdies nur wenig befriedigende Ausbeuten (etwa 26%, bezogen auf Toluol) liefert.

Nach dem Vorschlag der US-PS 4 042 600 stellt man I durch Diazotierung eines Anthranilsäureesters (II), Umsetzung der wässrigen Lösung des Diazoniumsalzes (III) mit Schwefeldioxid in Gegenwart von Kupfer-I-chlorid und Essigsäure zum entsprechenden Benzoesäureester-o-sulfochlorid (IV), Pyrolyse von IV zum o-Sulfobenzoesäureanhydrid (V) und Umsetzung von V mit Ammoniak her, wie durch folgendes Reaktionsschema veranschaulicht wird:

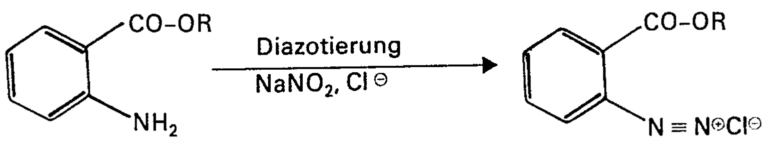


II III

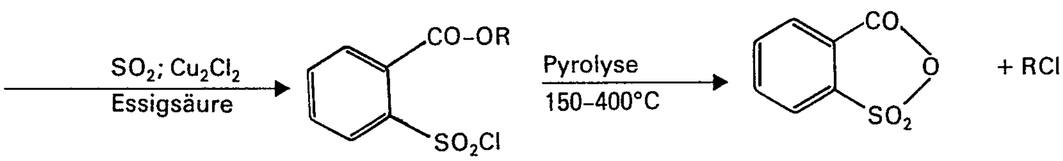


IV V

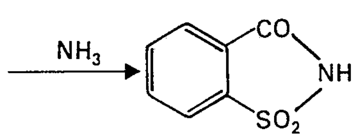


R = org. Rest

Auch dieser Weg ist indes umständlich, weil er u.a. die Isolierung der Zwischenstufen IV und V voraussetzt und weil die technisch ohnehin schwierig zu beherrschende Pyrolyse unvollständig ist. Ferner kann man die Essigsäure mit wirtschaftlich vertretbarem Aufwand nicht wieder zurückgewinnen.

Die Umsetzung von Aryldiazoniumchloriden zu den entsprechenden Sulfochloriden analog des Reaktionsschrittes III→IV wurde bereits früher von Meerwein et al. beschrieben (Chem. Ber., Band 90, 1957, Seite 841 ff), wobei ein Zusatz eines wasserunlöslichen Lösungsmittels mit kleiner Dielektrizitätskonstante wie Tetrachlorkohlenstoff und Benzol zur Erhöhung der Ausbeute empfohlen wurde. Gleichwohl sind die Ausbeuten bei dieser Arbeitsweise, die an den Beispielen des p-Methoxybenzol-sulfochlorids und des ß-Naphthalin-sulfochlorids erläutert wurde, mit jeweils rd. 40% unzureichend.

Weiterhin ist aus der DD-PS 4564 eine Saccharin-Synthese bekannt, welche ebenfalls über die Diazotierung eines Anthranilsäureesters und die Umsetzung des Diazoniumsalzes mit $SO_2$ verläuft. Dieses Verfahren ist jedoch sowohl wirtschaftlich als auch verfahrenstechnisch evident nachteilig, weil man die Diazotierung zur Vermeidung grösserer Wasservolumina mit nitrosen Gasen und die weitere Umsetzung mit flüssigem $SO_2$ vornehmen muss.

In Anbetracht der bisher bekannt gewordenen unbefriedigenden Synthesen des Saccharins lag der Erfindung die Aufgabe zugrunde, diese Verbindung auf einfachere und wirtschaftlichere Weise herzustellen.

Demgemäss wurde gefunden, dass man Saccharin auf dem Wege über die Umsetzung einer wässrig-salzsauren o-Methoxycarbonyl-benzoldiazoniumchlorid-Lösung mit Schwefeldioxid in einer bemerkenswerten Reaktionsfolge erhält, wenn man

a) die Diazoniumsalzlösung bei 0–100°C in Gegenwart eines mit Wasser nicht oder nur begrenzt mischbaren inerten organischen Lösungsmittels mit Schwefeldioxid reagieren lässt,

b) zur Zersetzung des Diazoniumsalzes gleichzeitig oder anschliessend einen Diazoniumsalz-Zersetzungskatalysator auf das Reaktionsgemisch einwirken lässt,
c) das wässrig-organische Reaktionsgemisch oder die organische Phase nach Abtrennung der wässrigen Phase bei 0–100°C mit einem Oxidationsmittel behandelt und
d) die organische Phase bei 0–50°C mit wässrigem Ammoniak umsetzt und aus der wässrigen Phase das Saccharin durch Ansäuern mit einer starken Säure in an sich bekannter Weise isoliert.

Die vor dem Reaktionsschritt (a) erforderliche Diazotierung des Anthranilsäuremethylesters ist an sich bekannt und bedarf daher keiner eingehenderen Erläuterung. Im Hinblick auf eine möglichst vollständige Diazotierung und im Hinblick auf das gute Gelingen des erfindungsgemässen Verfahrens hat es sich jedoch als besonders zweckmässig erwiesen, die Diazotierung in salzsaurem Medium mittels Natriumnitrit bei –10 bis 10°C vorzunehmen, wobei man pro Mol des Anthranilsäureesters 1,5 bis 4 mol HCl und 0,2 bis 1 kg Wasser verwendet.

Im Verfahrensschritt (a) lässt man die wässrigsaure, vorzugsweise 10 bis 40 Gew.-% des Diazoniumsalzes enthaltende Lösung in Gegenwart eines mit Wasser nicht oder nur begrenzt mischbaren inerten organischen Lösungsmittels mit Schwefeldioxid reagieren.

Zur Erzielung möglichst hoher Ausbeuten an dem Reaktionsprodukt verwendet man zweckmässigerweise mindestens äquimolare Mengen $SO_2$, bezogen auf das Diazoniumsalz. jedoch empfiehlt es sich im allgemeinen, einen molaren $SO_2$-Überschuss bis zu 1 mol, vorzugsweise von etwa 0,1 bis 0,3 mol, einzusetzen. Die Menge des Lösungsmittels beträgt je nach dessen Lösevermögen für das $SO_2$ und das entstehende Sulfochlorid vorzugsweise 0,1 bis 1,1 l pro Liter der wässrigen Diazoniumsalzlösung.

Die Lösungsmittel können im Prinzip beliebig sein, sofern sie mit Wasser nicht nennenswert mischbar sind, sich gegen wässrige Säuren und $SO_2$ im wesentlichen inert verhalten und ein ausreichendes Lösevermögen für $SO_2$ und das entstehende Sulfochlorid aufweisen.

Die gute Löslichkeit für das Sulfochlorid ist von besonderer Wichtigkeit, so dass sich die Wahl des Lösungsmittels in erster Linie hiernach richtet. Bei dem Erfordernis der geringen Wasserlöslichkeit genügt es dagegen im Prinzip, dass sich eine organische Phase ausbildet. Aus verfahrenstechnischen Gründen ist es jedoch vorteilhaft, ein möglichst wasserunlösliches Lösungsmittel zu verwenden, weil dadurch die im Verfahrenskreislauf erforderliche Trennung des Lösungsmittels von der wässrigen Phase erleichtert wird.

Geeignete Lösungsmittel sind beispielsweise

- aliphatische Ether mit 4 bis 20 C-Atomen wie Diethylether, Di-n-propylether, Diisopropylether, Methylethylether und Methyl-tert.-butylether
- aliphatische Alkohole mit 4 bis 8 C-Atomen wie n-Butanol, n-Pentanol und die Hexanole
- aliphatische Ester mit 2 bis 10 C-Atomen wie Methylacetat, Ethylacetat, n-Propylacetat, Isopropylacetat und die Butylacetate
- aliphatische Ketone mit 4 bis 10 C-Atomen wie Methylethylketon, Diethylketon, Methylisopropylketon und Diisopropylketon
- chlorierte aliphatische Kohlenwasserstoffe mit 1 bis 4 C-Atomen wie Methylenchlorid und die Dichlor- und Trichlorethane und -propane, wobei allgemein solche bevorzugt werden, die bis zu zwei Cl-Atome pro C-Atom enthalten
- aromatische Kohlenwasserstoffe mit 6 bis 10 C-Atomen wie Benzol, Toluol und die Xylole
- chlorierte aromatische Kohlenwasserstoffe wie Chlorbenzol und die Dichlor- und Trichlorbenzole
- Nitrile wie Benzonitril
- Nitroverbindungen wie Nitrobenzol

sowie Gemische dieser Lösungsmittel.

Aus wirtschaftlichen und verfahrenstechnischen Gründen werden allgemein solche Lösungsmittel bevorzugt, die bei Normaldruck zwischen 50 und 150°C sieden, da diese bei den im Rahmen des technisch ausgeübten Verfahrens erforderlichen Destillationsschritten am wenigsten Energie verbrauchen. Unter den genannten Gruppen von Lösungsmitteln empfehlen sich besonders die Ketone und die chlorierten aliphatischen Kohlenwasserstoffe, wogegen die aromatischen Lösungsmittel sich als etwas weniger geeignet erwiesen haben.

Man kann die Umsetzung in der Weise vornehmen, dass man eine Lösung aus dem Lösungsmittel und Schwefeldioxid mit der wässrigen Diazoniumsalzlösung oder die drei Komponenten $-SO_2$, Lösungsmittel, wässrige Phase – intensiv miteinander vermischt. In beiden Fällen empfehlen sich Temperaturen von 10–80°C sowie Normaldruck oder ein leicht erhöhter Druck bis zu etwa 6 bar.

Im Verfahrensschritt (b) wird das intermediäre Reaktionsprodukt des Verfahrensschrittes (a) mit Hilfe von Diazoniumsalz–Zersetzungskatalysatoren bei vorzugsweise 20 bis 80°C, besonders 40 bis 60°C, unter Stickstoffentwicklung in dem zweiphasigen Reaktionsgemisch des Verfahrensschrittes (a) zersetzt.

Als Zersetzungskatalysatoren kommen z.B. Kupfer und Wolfram sowie deren Verbindungen in Betracht, die man vorzugsweise in Mengen von 0,01 bis 0,1, besonders 0,02 bis 0,03 mol pro Mol des Anthranilsäuremethylesters in fester Form oder, falls die Katalysatoren wasserlöslich sind, in Form konzentrierter wässriger Lösungen einsetzt. Besonders bevorzugt werden $CuCl_2$ und $Cu_2Cl_2$ als Katalysatoren.

Überraschenderweise eignen sich auch oberflächenaktive quartäre Ammoniumsalze als Zersetzungskatalysatoren in Mengen von vorzugsweise etwa 0,01 bis 0,1 mol pro Mol des Anthranilsäuremethylesters. Als besonders geeignet haben sich quartäre Ammoniumsalze des Typs

$R{-}N^{\oplus}R'_3 \cdot X^{\ominus}$ erwiesen, wobei ein langkettiger Alkylrest, die Reste R′ niedere Alkyl- oder Hydroxyalkylgruppen wie die Methyl- oder Hydroxyethylgruppe und $X^{\ominus}$ ein Anionenäquivalent wie vorzugsweise Chlorid bedeuten. Beispiele für derartige Verbindungen sind Lauryltrimethylammoniumchlorid und Stearyltrimethylammoniumchlorid.

Die Oberflächenaktivität der quartären Ammoniumsalze braucht im übrigen nicht besonders ausgeprägt zu sein. Vielmehr genügt es, wenn diese Salze hinreichend organophil sind wie beispielsweise Tetrabutylammoniumsalze oder Trimethylbenzylammoniumsalze.

Verwendet man Kupfersalze als Zersetzungskatalysatoren, so bewirken oberflächenaktive Verbindungen ganz allgemein eine Beschleunigung der Zersetzung und damit eine Erhöhung der Raum-Zeit-Ausbeute. Die Mengen an diesen Mitteln, die anionisch, nicht-ionisch oder kationisch sein können, betragen vorzugsweise 0,01 bis 0,1 mol pro Mol des Anthranilsäuremethylesters. Auch in diesem Falle empfehlen sich die kationisch oberflächenaktiven quartären Ammoniumsalze, da sie für sich allein bereits als Zersetzungskatalysatoren fungieren, wobei es von besonderem Vorteil ist, dass nur noch etwa ein Zehntel der Menge an Kupfersalzen erforderlich ist, die man zur Erzielung der gleichen Wirkung mit den Kupfersalzen allein benötigt.

Mit dem freigesetzten Stickstoff entweicht auch ein Teil des Schwefeldioxids, welches in üblicher Weise aus dem Abgasstrom entfernt und wieder in die Verfahrensstufe (b) zurückgeführt werden kann, z.B. durch Wäsche mit dem Lösungsmittel. Der Anteil des zurückgewinnbaren $SO_2$ kann erhöht werden, indem man es nach beendeter Reaktion, also wenn kein Stickstoff mehr entwickelt wird, durch Erwärmen aus dem Reaktionsgemisch austreibt.

In einer alternativen Ausführungsform kann man die Umsetzung mit dem $SO_2$ und dem Katalysator auch gleichzeitig vornehmen. Diese Methode ist in der Regel zu bevorzugen, da sich hierdurch die Raum-Zeit-Ausbeute erhöhen lässt und da wegen kürzerer Gesamtreaktionszeiten weniger Nebenprodukte gebildet werden.

Als Reaktionsprodukt des Verfahrensschrittes (b) erhält man ein wässrig-organisches Gemisch, dessen organische Phase das Benzoesäuremethylester-o-sulfochlorid enthält.

Im Verfahrensschritt (c) lässt man auf dieses Gemisch bei 0 bis 100, vorzugsweise 20 bis 50°C und bei Normaldruck oder leicht erhöhtem Druck – etwa bis 6 bar – ein Oxidationsmittel einwirken. Diese Massnahme beruht auf der Beobachtung, dass sich im Reaktionsschritt (b) als Nebenprodukt die Benzoesäuremethylester-o-sulfinsäure bildet. Überraschenderweise lässt sich die Sulfinsäure ohne weiteres wieder zum Sulfochlorid oxidieren, so dass sich die Ausbeute am Sulfochlorid erheblich erhöhen lässt. Im Prinzip eignet sich jedes Oxidationsmittel, jedoch bevorzugt man aus verfahrenstechnischen Gründen Wasserstoffperoxid und vor allem Chlorgas. Die zweckmässige Menge des Oxidationsmittels beträgt etwa 0,1 bis 0,5 mol, in der Regel 0,2 bis 0,3 mol pro Mol eingesetztem Diazoniumsalz. Die Sulfinsäure kann nach der Methode der Hochdruckflüssigkeitschromatographie (HPLC) nachgewiesen werden. In der Regel ist die Oxidation nach etwa 5 bis 30 Minuten beendet.

Anschliessend trennt man die wässrige von der organischen Phase. Die wässrige Phase wird, nachdem man sie in üblicher Weise unter Rückgewinnung des Zersetzungskatalysators aufgearbeitet hat, verworfen.

Man kann die wässrige Phase auch von der oxidativen Behandlung abtrennen, jedoch empfiehlt es sich in der Regel, diesen Schritt an die Oxidation anzuschliessen, weil die wässrige Phase die wasserlöslichen Nebenprodukte wie u.a. Salze und die restliche Sulfinsäure aufnimmt.

Im Verfahrensschritt (d) wird die nach (c) erhaltene organische Phase, welche das Benzoesäuremethylester-o-sulfochlorid enthält, bei 0 bis 50°C, vorzugsweise 20 bis 40°C mit wässrigem Ammoniak umgesetzt. Die Menge des Ammoniaks, den man vorzugsweise in Form seiner 10 bis 25 gew.-%igen wässrigen Lösungen einsetzt, beträgt vorzugsweise 3 bis 4, besonders 3,2 bis 3,5 mol $NH_3$ pro Mol des Sulfochlorids.

Das sich bei dieser Umsetzung bildende Saccharin wird von der wässrigen Phase aufgenommen, aus der es durch Ansäuern mit einer starken Säure wie Salzsäure oder Schwefelsäure wie üblich ausgefällt wird. Die weitere Reinigung des Saccharins kann dann in bekannter Weise erfolgen, z.B. durch erneute Umfällung. Weiterhin kann man das Saccharin auch, ebenfalls wie üblich, in sein Natriumsalz überführen. Die bei der Saccharinbildung zurückbleibende organische Phase kann, gegebenenfalls nach einer destillativen Zwischenreinigung, wieder in den Verfahrenskreislauf zurückgeführt werden.

Man kann das erfindungsgemässe Verfahren diskontinuierlich oder nach an sich bekannten Techniken kontinuierlich ausführen. Da es sich bis auf die Saccharinausfällung um Reaktionen in flüssigen Phasen handelt, lässt sich die kontinuierliche Arbeitsweise verfahrenstechnisch besonders einfach und wirtschaftlich realisieren, verglichen mit den bisherigen Saccharin-Synthesen.

Berechnet auf den eingesetzten Anthranilsäuremethylester erhält man das Rohsaccharin, welches bereits in einer Reinheit von über 99% anfällt, in Ausbeute von 90% und darüber.

Beispiel 1

196 g 30 gew.-%ige wässrige Salzsäure (= 1,61 mol HCl) wurden im Laufe von 30 min bei -5 bis +10°C allmählich mit 76 g (0,5 mol) Anthranilsäuremethylester und 135 g einer wässrigen Natriumnitritlösung (= 0,5 mol $NaNO_2$) versetzt. Danach wurde die Diazoniumsalzlösung bei 20°C unter intensivem Rühren 10 min lang mit einer Lösung aus 200 ml 1,2-Dichlormethan und 42 g (0,65 mol) $SO_2$ in Kontakt gebracht.

Nach Zugabe von 5 g einer wässrigen $CuCl_2$-

Lösung (Cu-Gehalt 0,9 g = 0,014 mol) wurde das Reaktionsgemisch auf 50°C erwärmt, wobei sich ein kräftiger $N_2$-Strom entwickelte. Dieser Verfahrensschritt nahm 80 min in Anspruch, wonach die wässrige Phase abgetrennt wurde. In die organische Phase wurden sodann bei 40°C im Laufe von 10 min 10 g (0,14 mol) Chlorgas eingeleitet, wonach das Gemisch noch 5 min lang gerührt wurde.

Das so erhaltene Gemisch wurde anschliessend unter kräftigem Rühren bei 30–40°C innerhalb von 5 min mit 250 g wässrigem Ammoniak ($NH_3$-Gehalt 33 g = 1,94 mol) versetzt, wonach die Phasen getrennt wurden. Aus der wässrigen Phase wurde das Saccharin schliesslich bei 30 bis 40°C mit 92 g wässriger Salzsäure (HCl-Gehalt 27 g = 0,74 mol) in Form eines weissen Kristallbreis ausgefällt.

Nach der üblichen Isolierung und Trocknung fiel das Saccharin in einer Reinheit (nach HPLC) von 99,5% an (Smp. 227–229°C. Die Ausbeute betrug 89,4%.

Beispiel 2

Auf analoge Weise wie in Beispiel 1, jedoch mit dem Unterschied, dass als organisches Lösungsmittel Methylisopropylketon diente, dass das $CuCl_2$ in fester Form zugegeben wurde und dass nur 5 g $Cl_2$ verwendet wurden, fiel das Saccharin in 99,6%iger Reinheit und in 90,8%iger Ausbeute an.

Beispiel 3

Auf analoge Weise wie in Beispiel 1, jedoch mit dem Unterschied, dass als Lösungsmittel Diethylketon diente und dass die $CuCl_2$-Lösung gleichzeitig mit der $SO_2$-Lösung zu der Diazoniumsalzlösung gegeben wurde, fiel das Saccharin in 99,8%iger Reinheit und in 92,4%iger Ausbeute an.

Beispiel 4

Auf analoge Weise wie in Beispiel 3, jedoch mit dem Unterschied, dass anstelle des Chlors die äquivalente Menge (rd. 2,5 g) Wasserstoffperoxid als Oxidationsmittel verwendet wurde, fiel das Saccharin in einer Reinheit von 99,6% und einer Ausbeute von 93,1% an.

Beispiel 5

Auf analoge Weise wie in Beispiel 1, jedoch mit 20 g (rd. 0,08 mol) Lauryltrimethylammoniumchlorid anstelle des Kupferchlorides, nahm die Zersetzung des Diazoniumsalzes etwa 100 min in Anspruch. Die Reinheit des Saccharins betrug in diesem Falle 98,7% und die Ausbeute 72%.

Beispiel 6

Auf analoge Weise wie in Beispiel 5, jedoch unter zusätzlicher Mitverwendung von 0,5 g der wässrigen $CuCl_2$-Lösung (= rd. 0,001 mol Cu) nahm die Zersetzung des Diazoniumsalzes nur 40 min in Anspruch. Die Reinheit des Saccharins betrug in diesem Falle 99,8% und die Ausbeute 92,7%.

**Patentansprüche**

1. Verfahren zur Herstellung von Saccharin auf dem Wege über die Umsetzung einer wässrig-salzsauren o-Methoxy-carbonylbenzoldiazoniumchlorid-Lösung mit Schwefeldioxid und einem Diazoniumsalz-Zersetzungskatalysator, dadurch gekennzeichnet, dass man

a) die wässrige Diazoniumsalzlösung bei 0 bis 100°C in Gegenwart eines mit Wasser nicht oder nur begrenzt mischbaren inerten organischen Lösungsmittels mit Schwefeldioxid reagieren lässt,
b) zur Zersetzung des Diazoniumsalzes gleichzeitig oder anschliessend einen Diazoniumsalz-Zersetzungskatalysator auf das Reaktionsgemisch einwirken lässt,
c) das wässrig-organische Reaktionsgemisch oder die organische Phase nach Abtrennung der wässrigen Phase bei 0 bis 100°C mit einem Oxidationsmittel behandelt und
d) die organische Phase bei 0 bis 50°C mit wässrigem Ammoniak umsetzt und aus der wässrigen Phase das Saccharin durch Ansäuern mit einer starken Säure in an sich bekannter Weise isoliert.

2. Verfahren nach Anspruch 1, dadurch gekennzeichnet, dass man als organisches Lösungsmittel ein aliphatisches Keton mit 4 bis 10 C-Atomen verwendet.

3. Verfahren nach Anspruch 1, dadurch gekennzeichnet, dass man als organisches Lösungsmittel einen chlorierten aliphatischen Kohlenwasserstoff mit 1 bis 4 C-Atomen verwendet, der bis zu zwei Cl-Atome pro C-Atom enthält.

4. Verfahren nach den Ansprüchen 1 bis 3, dadurch gekennzeichnet, dass man als Diazoniumsalz-Zersetzungskatalysator $CuCl_2$ oder $Cu_2Cl_2$ verwendet.

5. Verfahren nach Anspruch 4, dadurch gekennzeichnet, dass man zur Zersetzung des Diazoniumsalzes zusätzlich zu den Kupfersalzen ein oberflächenaktives Mittel mitverwendet.

6. Verfahren nach Anspruch 5, dadurch gekennzeichnet, dass man als oberflächenaktives Mittel ein oberflächenaktives quartäres Ammoniumsalz verwendet.

7. Verfahren nach den Ansprüchen 1 bis 3, dadurch gekennzeichnet, dass man als Diazoniumsalz-Zersetzungskatalysator ein oberflächenaktives quartäres Ammoniumsalz verwendet.

8. Verfahren nach den Ansprüchen 1 bis 7, dadurch gekennzeichnet, dass man als Oxidationsmittel Chlorgas verwendet.

9. Verfahren nach den Ansprüchen 1 bis 7, dadurch gekennzeichnet, dass man als Oxidationsmittel Wasserstoffperoxid verwendet.

**Claims**

1. A process for the preparation of saccharin by reacting an aqueous hydrochlorid acid solution of o-methoxy-carbonylbenzenediazonium

chloride with sulfur dioxide and a diazonium salt decomposition catalyst, wherein

a) the aqueous diazonium salt solution is reacted with sulfur dioxide at from 0 to 100°C in the presence of a water-immiscible or only partially water-miscible inert organic solvent,

b) in order to decompose the diazonium salt, the reaction mixture is treated simultaneously or subsequently with a diazonium salt decomposition catalyst,

c) the aqueous organic reaction mixture, or the organic phase obtained after removing the aqueous phase, is treated with an oxidizing agent at from 0 to 100°C, and

d) the organic phase is reacted with aqueous ammonia at from 0 to 50°C and the saccharin is isolated from the aqueous phase in a conventional manner by acidifying with a strong acid.

2. A process as claimed in claim 1, wherein the organic solvent used is an aliphatic ketone of 4 to 10 carbon atoms.

3. A process as claimed in claim 1, wherein the organic solvent used is an aliphatic chlorohydrocarbon of 1 to 4 carbon atoms, containing up to two chlorine atoms per carbon atom.

4. A process as claimed in claims 1 to 3, wherein $CuCl_2$ or $Cu_2Cl_2$ is used as the diazonium salt decomposition catalyst.

5. A process as claimed in claim 4, wherein a surfactant is used conjointly with the copper salts to decompose the diazonium salt.

6. A process as claimed in claim 5, wherein a surface-active quaternary ammonium salt is used as the surfactant.

7. A process as claimed in claims 1 to 3, wherein a surface-active quaternary ammonium salt is used as the diazonium salt decomposition catalyst.

8. A process as claimed in claims 1 to 7, wherein chlorine gas is used as the oxidizing agent.

9. A process as claimed in claims 1 to 7, wherein hydrogen peroxide is used as the oxidizing agent.

**Revendications**

1. Procédé pour la préparation de saccharine par la voie passant par la mise en réaction d'une solution chlorhydride-aqueuse de chlorure d'o-méthoxycarbonylbenzène-diazonium avec le bioxyde de soufre et un catalyseur de décomposition des sels de diazonium, caractérisé en ce que

a) l'on fait réagir la solution aqueuse du sel de diazonium avec le bioxyde de soufre à une température de 0 à 100°C, en présence d'un solvant organique inerte, non miscible ou seulement miscible de façon limitée à l'eau,

b) l'on fait agir, en même temps ou immédiatement après, un catalyseur de décomposition des sels de diazonium sur le mélange réactionnel, afin de décomposer le sel de diazonium,

c) l'on traite le mélange réactionnel organique-aqueux ou la phase organique après séparation de la phase aqueuse, par un agent d'oxydation à une température de 0 à 100°C, et

d) l'on fait réagir la phase organique avec l'ammoniac aqueux à une température de 0 à 50°C et on isole la saccharine de la phase aqueuse, de façon connue en soi, par acidification avec un acide fort.

2. Procédé selon la revendication 1, caractérisé en ce qu'on utilise, en tant que solvant organique, une cétone aliphatique à 4–10 atomes de C.

3. Procédé selon la revendication 1, caractérisé en ce qu'on utilise, en tant que solvant organique, un hydrocarbure aliphatique chloré à 1–4 atomes de C qui contient jusqu'à deux atomes de Cl par atome de C.

4. Procédé selon l'une quelconque des revendications 1 à 3, caractérisé en ce qu'on utilise $CuCl_2$ ou $Cu_2Cl_2$ en tant que catalyseur de décomposition des sels de diazonium.

5. Procédé selon la revendication 4, caractérisé en ce qu'on utilise simultanément, pour la décomposition du sel de diazonium, un agent tensio-actif en plus des sels de cuivre.

6. Procédé selon la revendication 5, caractérisé en ce qu'on utilise, en tant qu'agent tensioactif, un sel d'ammonium quaternaire tensioactif.

7. Procédé selon l'une quelconque des revendications 1 à 3, caractérisé en ce qu'on utilise, en tant que catalyseur de décomposition des sels de diazonium, un sel d'ammonium quaternaire tensio-actif.

8. Procédé selon l'une quelconque des revendications 1 à 7, caractérisé en ce qu'on utilise le gaz chlore comme agent d'oxydation.

9. Procédé selon l'une quelconque des revendications 1 à 7, caractérisé en ce qu'on utilise le peroxyde d'hydrogène comme agent d'oxydation.